# EUROPÄISCHE PATENTSCHRIFT

(11) **EP 3 494 850 B1**
(45) Veröffentlichungstag und Bekanntmachung des Hinweises auf die Patenterteilung: **13.04.2022**
(21) Anmeldenummer: 18210893.6
(22) Anmeldetag: 06.12.2018
(51) Int. Cl.: A47K 10/16, A61K 8/35, A61Q 17/00, A61Q 19/00, A61Q 19/08, C11D 3/386, C11D 17/04, D06M 16/00

(54) **FEUCHTTUCH MIT HYDROXYACETOPHENON**
WET WIPE WITH HYDROXYACETOPHENON
LINGETTE HUMIDE AVEC HYDROXYACETOPHENON

(30) Priorität: 11.12.2017 DE 102017129498
(43) Veröffentlichungstag der Anmeldung: 12.06.2019
(73) Patentinhaber: Dr. Schumacher GmbH, 34323 Malsfeld-Beiseförth (DE)
(72) Erfinder: SCHUMACHER, Sönke, 34323 Malsfeld (DE); BALLEZ, Mike, 34323 Malsfeld (DE); NIELSEN, Jens, 34323 Malsfeld (DE)
(74) Vertreter: Sandvoß, Stefanie

(56) Entgegenhaltungen:
- DE-A1-102007 031 661
- DE-U1-202017 105 640

## Beschreibung

Feuchttücher sind bekanntermaßen Vliesstoffe, die mit einer in der Regel flüssigen Zubereitung getränkt sind, die u.a. einen reinigenden und/oder pflegenden Inhaltsstoff enthält. Feuchttücher finden mittlerweile in vielen Bereichen der persönlichen Pflege und vor allem Babypflege Anwendung und erfreuen sich sehr hoher Beliebtheit, da sie die Möglichkeit einer schnellen und bequemen Reinigung bzw. Pflege bieten, ohne dass dafür zusätzliches Wasser erforderlich ist. In der Regel zu handlichen Einheiten von ca. 10 bis 100 Tüchern verpackt, zeichnen sich Feuchttücher darüber hinaus auch durch ihre Portabilität aus und sind vor allem auch aufgrund dieser Eigenschaft aus unserer immer mobiler werdenden Gesellschaft nicht mehr wegzudenken.

Doch Feuchttücher sind auch ein ausgesprochen gutes Nährmedium für das Wachstum von Pilzen, Bakterien und Hefen, die sich nach dem Eindringen von Mikroorganismen infolge des wiederholten Öffnens und Schließens der Packung bei der darin befindlichen Feuchtigkeit und z.B. bei Zimmertemperatur rasant vermehren. Es ist daher eine Herausforderung für die Hersteller von Feuchttüchern, das unerwünschte Wachstum von Pilzen, Bakterien und Hefen zu unterbinden und auch einige Zeit nach Anbruch einer Packung mit Feuchttüchern eine Keimfreiheit der Tücher zu gewährleisten.

Zur Hemmung bzw. Verhinderung des Wachstums von Mikroorganismen in angebrochenen Packungen mit Feuchttüchern enthalten die kosmetischen Zubereitungen, mit denen die Vliesstoffe getränkt sind, daher notwendigerweise Verbindungen mit konservierenden Eigenschaften.

Als Konservierungsstoffe für kosmetische Mittel, zu denen auch Tränkflüssigkeiten für Feuchttücher zählen, sind EU-weit derzeit nur etwa 50 Stoffe zugelassen, die zuvor einer umfangreichen Sicherheitsprüfung unterzogen und als sicher beurteilt wurden.

Oftmals enthalten Feuchttücher jedoch auch sog. "multifunktionelle Additive" als Inhaltsstoffe, die keine reinen Konservierungsstoffe sind, sondern neben einer ersten Eigenschaft, beispielsweise einer emulgierenden, feuchtigkeitsspendenden oder rückfettenden Wirkung, als weitere Eigenschaft z.B. auch eine konservierende Wirkung aufweisen. Ein solches multifunktionelles Additiv ist beispielsweise Hydroxyacetophenon, das im Stand der Technik für seine stabilisierenden Eigenschaften bekannt ist oder in Kombination mit einem Zimtsäurederivat oder herkömmlichen Konservierungsmitteln in kosmetischen Zubereitungen eingesetzt wird.

Die EP 2 774 481 A1 beschreibt die Eignung von Hydroxyacetophenon und ausgewählten Derivaten als antimikrobielle Wirkstoffe, die in Kombination mit weiteren antimikrobiellen Wirkstoffen u.a. zur Konservierung von Feuchttüchern eingesetzt werden.

Die WO 2017/093428 A1 beschreibt kosmetische Zusammensetzungen, die eine Kombination aus Hydroxyacetophenon und 1,6-Hexandiol als Konservierungsmittel enthalten.

Die CN 107432834 A beschreibt eine kosmetische Zusammensetzung mit antibakteriellen Eigenschaften, die neben Hydroxyacetophenon noch Glyceryl Caprylate, Caprylohydroxamsäure, Octylglycol und Ethylhexylglycerin als Verbindungen mit konservierenden Eigenschaften enthält.

Die DE 10 2014 104 253 A1 beschreibt den Einsatz von 4-Hydroxyacetophenon als Stabilisator in Wasser-in-Öl-Emulsionen. Diese sollen mit 4-Hydroxyacetophenon als Additiv im Laufe der Lagerzeit einen geringeren Viskositätsabfall aufweisen als mit anderen niedermolekularen Additiven wie Methylparaben oder Phenoxyethanol.

Die DE 10 2014 104 254 A1 beschreibt kosmetische oder dermatologische Zubereitungen, die als Wirkstoffkombination 4-Hydroxyacetophenon und zumindest ein Zimtsäurederivat enthalten. Insbesondere sollen sich die diese Wirkstoffkombination enthaltenden Zubereitungen als Mittel in der Behandlung und Prophylaxe von Hautalterung eignen, die durch oxidative Prozesse hervorgerufen wird.

Die DE 10 2007 031 661 A1 beschreibt Selbstbräunungszusammensetzungen, die auch zur Applikation auf Haaren geeignet sein sollen, um die Sichtbarkeit grauer Haare oder eines Haaransatzes zu verringern. Die Selbstbräunungszusammensetzungen enthalten Dihydroxyaceton und/oder Erythrulose sowie mindestens einen Enhancer aus der Gruppe der Hexahydroxycyclohexane und/oder Acetyl-Methionyl-Arginin-Ethylester. Weiterhin kannHydroxyacetophenon in der Zusammensetzung enthalten sein und die Zusammensetzung soll auch zur Tränkung von Feuchttüchern geeignet sein. Die DE 20 2017 105 640 U1 beschreibt schweißhemmende und/oder Schweißgeruch hemmende kosmetische oder pharmazeutische Zubereitungen, die Aluminiumlactat sowie mindestens einen Pflanzenextrakt enthalten. Optional können auch Konservierungsmittelhelfer. z.B. Hydroxyacetophenon, enthalten sein, und die Zubereitung soll auch auf ein wegwerfbares Substrat, z B ein Feuchtluch, aufgebracht werden können.

### Aufgabe der Erfindung

Es ist die Aufgabe der vorliegenden Erfindung, ein alternatives Feuchttuch bereitzustellen, das sich vorzugsweise durch einen geringen Gehalt an konservierenden Inhaltsstoffen in dem enthaltenen Tränkungsmittel auszeichnet, durch welchen jedoch das Wachstum von Mikroorganismen in erheblichem Maße gehemmt bzw. ganz verhindert wird.

### Allgemeine Beschreibung der Erfindung

Die Erfindung löst diese Aufgabe mit den Merkmalen der Ansprüche und mit einem Feuchttuch, das einen Vliesstoff und ein mehrere Inhaltsstoffe enthaltendes Tränkungsmittel umfasst, wobei das Tränkungsmittel Hydroxyacetophenon als alleinige Verbindung mit konservierenden Eigenschaften enthält.

Für die Zwecke der Erfindung wird unter einem Feuchttuch ein Vliesstoff verstanden, der mit einer flüssigen oder halbflüssigen Zusammensetzung kontaktiert bzw. getränkt wurde, unabhängig davon, ob diese flüssige oder halbflüssige Zusammensetzung wasser- oder ölbasiert ist. Insbesondere werden daher auch Öltücher als Feuchttücher verstanden. Weiterhin werden insbesondere auch nicht rechteckige Vliesstoffe, beispielsweise runde oder ovale Pads, die mit einer flüssigen oder halbflüssigen Zusammensetzung kontaktiert bzw. getränkt wurden, für die Zwecke der Erfindung als Feuchttuch bezeichnet.

Unter Hydroxyacetophenon wird jede Verbindung verstanden, die unter Formel (**I**) fällt:

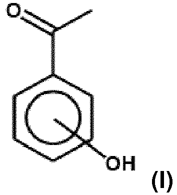

Hydroxyacetophenon kann daher für die Zwecke der Erfindung 2-Hydroxyacetophenon, 3-Hydroxyacetophenon, 4-Hydroxyacetophenon oder eine Mischung aus 2-Hydroxyacetophenon und/oder 3-Hydroxyacetophenon und/oder 4-Hydroxyacetophenon sein.

Es hat sich überraschend gezeigt, dass Hydroxyacetophenon, das im Stand der Technik jeweils nur in Kombination mit zumindest einem herkömmlichen Konservierungsmittel zur Konservierung von kosmetischen Zusammensetzungen bekannt ist, als alleiniges Konservierungsmittel sogar für Feuchttücher geeignet ist. Zusätzlich zu dieser an sich schon überraschenden Eignung von Hydroxyacetophenon als alleinigem Inhaltsstoff mit konservierenden Eigenschaften in kosmetischen Zusammensetzungen ist dabei insbesondere die hervorragende antimikrobielle Wirkung erstaunlich, die das in einem Tränkungsmittel enthaltene Hydroxyacetophenon auf ein Feuchttuch aufgebracht entfaltet. Denn Feuchttücher begünstigen aufgrund ihrer großen Oberfläche und den in einer angebrochenen Packung vorherrschenden Bedingungen, d.h. insbesondere im feucht-warmem Milieu und in Kontakt mit Luftsauerstoff, naturgemäß das Wachstum von Pilzen, Bakterien und Hefen, welches ohne einen in dem Tränkungsmittel enthaltenen Konservierungsstoff sehr schnell zum mikrobiellen Verderb der gesamten Packung führen würde.

Ein erfindungsgemäßes Feuchttuch, das einen mit einem Tränkungsmittel versehenen Vliesstoff umfasst oder daraus besteht, wobei das Tränkungsmittel ausschließlich Hydroxyacetophenon als Verbindung mit konservierenden Eigenschaften enthält, zeichnet sich jedoch durch eine exzellente antimikrobielle Haltbarkeit aus, durch die ein unerwünschtes Wachstum von Mikroorganismen auf dem Tuch gehemmt und vorzugsweise gänzlich verhindert wird.

Gemäß einer Ausführungsform besteht der in einem erfindungsgemäßen Feuchttuch enthaltene Vliesstoff aus Viskosefasern und/oder Lyocell-Fasern und/oder PET-Fasern, d.h. er kann entweder zu 100 % aus Viskosefasern, Lyocell-Fasern oder PET-Fasern bestehen, oder aus Mischfasern dieser bestehen.

Optional enthält der Vliesstoff auch Mikrofasern und/oder Baumwoll-Fasern, beispielsweise zu einem Anteil von jeweils bis zu 20 Gew.-%, insbesondere von 0,1 - 5,0 Gew.-%.

Gemäß einer bevorzugten Ausführungsform besteht der in dem erfindungsgemäßen Feuchttuch enthaltene Vliesstoff aus Viskosefasern und/oder Lyocell-Fasern und/oder PET-Fasern und zusätzlich aus 0,1 - 20,0 Gew.-% Mikrofasern und/oder 0,1 - 20,0 Gew.-% Baumwoll-Fasern.

Besonders bevorzugt umfasst oder besteht der Vliesstoff eines erfindungsgemäßen Feuchttuches aus 30 - 70 Gew.-% Viskosefasern und 30 - 70 Gew.-% PET-Fasern sowie optional 0,1 - 5,0 Gew.-% Mikrofasern und/oder 0,1 - 30,0 Gew.-% Baumwoll-Fasern.

In Ausführungsformen, in denen der Vliesstoff weder Lyocell-Fasern, noch Mikrofasern oder Baumwoll-Fasern enthält, sondern ausschließlich aus Viskosefasern und PET-Fasern besteht, kann der Anteil von Viskosefasern zu PET-Fasern stufenlos variieren, beispielsweise von 1 Gew.-% Viskosefasern / 99 Gew.-% PET-Fasern zu 99 Gew.-% Viskosefasern / 1 Gew.-% PET-Fasern.

Die hervorragende antimikrobielle Wirkung eines Tränkungsmittels, das nur Hydroxyacetophenon als Verbindung mit konservierenden Eigenschaften enthält, konnte dabei auf allen vorgenannten Vliesstoffen bzw. Vliesstoffzusammensetzungen gezeigt werden.

Das Tränkungsmittel, mit dem der Vliesstoff in einem erfindungsgemäßen Feuchttuch getränkt ist, ist erfindungsgemäß frei von organischen Säuren, die im Kosmetikbereich z.B. als Konservierungsmittel eingesetzt werden. Organische Säuren dissoziieren jedoch, wenn der pH-Wert nicht richtig eingestellt ist, sodass kosmetische Zusammensetzungen, zu denen auch Tränkungsmittel für Feuchttücher zählen, in der Regel auch einen Puffer enthalten, wenn sie eine organische Säure als Inhaltstoff aufweisen, wobei durch den Puffer der pH-Wert der kosmetischen Zusammensetzung im Wesentlichen konstant gehalten wird.

Gegenüber herkömmlichen Feuchttüchern bietet ein erfindungsgemäßes Feuchttuch, dessen Tränkungsmittel frei von organischen Säuren und daher ungepuffert ist, den Vorteil, dass mit Hydroxyacetophenon als alleinigem Konservierungsmittel eine pH-unabhängige Konservierung von Feuchttüchern möglich ist bzw. der pH-Wert des Tränkungsmittels beliebig eingestellt und an die Bedürfnisse der zu kontaktierenden Hautpartien angepasst werden kann. Denn je nach Anwendungsbereich variieren die Bedürfnisse der Haut insbesondere hinsichtlich des pH-Wertes. Es ist daher äußerst vorteilhaft, dass das in einem erfindungsgemäßen Feuchttuch enthaltene Tränkungsmittel aufgrund des Verzichtes auf organische Säuren auf jeden beliebigen pH-Wert eingestellt werden kann, der für eine gewünschte Anwendung geeignet ist.

Vorzugsweise enthält das in einem erfindungsgemäßen Feuchttuch auf einen Vliesstoff aufgebrachte Tränkungsmittel ein Tensidsystem, das Betaine und/oder Lauryl Glucosid umfasst oder daraus besteht.

Der konservierende Inhaltsstoff Hydroxyacetophenon ist bevorzugt bis zu 1 Gew.-% in dem Tränkungsmittel enthalten, insbesondere zu 0,125 Gew.-% bis 0,6 Gew.-% und besonders bevorzugt zu etwa 0,25 Gew.-%. Dabei entfaltet das Hydroxyacetophenon sogar bei einer vergleichsweise geringen Tränkmenge des Vliesstoffes, beispielsweise von max. 1,0 g,
2,0 g, 3,0 g oder 4,0 g Tränkungsmittel pro Gramm Vliesstoff, eine sehr gute antimikrobielle Wirkung. Gegenüber herkömmlichen Feuchttüchern zeichnet sich das erfindungsgemäße Feuchttuch daher bevorzugt durch einen geringeren Bedarf an Tränkungsmittel bei vergleichbaren oder sogar verbesserten konservierenden Eigenschaften aus, wobei der geringere Bedarf an Tränkungsmittel natürlich auch eine erhebliche Kostenersparnis in der Tuchproduktion mit sich bringt.

Ein bevorzugtes erfindungsgemäßes Feuchttuch zeichnet sich daher durch eine Tränkmenge von max. 1,0 g bis 4,0 g Tränkungsmittel pro Gramm Vliesstoff aus, wobei das Tränkungsmittel 0,125 Gew.-% bis 0,6 Gew.-% und insbesondere etwa 0,25 Gew.-% Hydroxyacetophenon sowie optional Betaine und/oder Lauryl Glucosid enthält.

Die Erfindung betrifft weiterhin auch ein Verfahren zur Herstellung eines erfindungsgemäßen Feuchttuches, bei dem ein Tränkungsmittel, das Hydroxyacetophenon als einzigen Inhaltsstoff mit konservierenden Eigenschaften enthält, auf einen Vliesstoff aufgebracht wird, beispielsweise mit einer Tränkungsmenge von 1,0 g Tränkungsmittel pro Gramm Vliesstoff.

Der in dem erfindungsgemäßen Verfahren eingesetzte Vliesstoff und das in dem erfindungsgemäßen Verfahren eingesetzte Tränkungsmittel sind so beschaffen wie voranstehend mit Bezug auf das erfindungsgemäße Feuchttuch beschrieben.

Dadurch, dass das in dem erfindungsgemäßen Feuchttuch enthaltene Tränkungsmittel mit Hydroxyacetophenon ein pH-unabhängiges Konservierungsmittel enthält und der pH-Wert des Tränkungsmittels daher beliebig eingestellt werden kann, eignet sich ein erfindungsgemäßes Feuchttuch für zahlreiche verschiedene Anwendungen der körperlichen Pflege. Die Erfindung betrifft daher auch die Verwendung eines erfindungsgemäßen Feuchttuches zur Gesichts- und/oder Körperreinigung, als Anti-Aging-Mittel, als Anti-Pollution-Mittel, als Gesichtsmaske, zur Make-up-Entfernung, als Toilettenpapier und/oder als Tuch zur Babyreinigung.

Da Hydroxyacetophenon darüber hinaus auch eine gute Verträglichkeit für Schleimhäute und Augen aufweist, betrifft die Erfindung außerdem auch die Verwendung eines erfindungsgemäßen Feuchttuches für die Kontaktierung und/oder Reinigung von Schleimhäuten und/oder Augenpartien.

### Genaue Beschreibung der Erfindung

Die Erfindung wird nun genauer anhand von Ausführungsbeispielen beschrieben.

### Beispiel 1: Konservierunasbelastunastest erfindunasaemäßer mit Öl-in-Wasser-Emulsion getränkter Feuchttücher

Die Prüfung auf eine ausreichende Konservierung der erfindungsgemäßen Feuchttücher erfolgt standardmäßig nach dem Konservierungsbelastungstest (KBT). Dieses Prüfverfahren (Ph.Eur.5.01.03) wird beispielsweise für Kosmetika angewandt, die während der Lagerung und Anwendung einer möglichen mikrobiellen Verkeimung unterliegen. Die kosmetische Zubereitung wird dabei mit einer definierten Menge an Mikroorganismen beimpft, um anschließend in definierten Zeitintervallen ein Aliquot zu entnehmen und die Anzahl der entsprechenden Mikroorganismen zu bestimmen. Aus dem Verhältnis der Keimzahlen nach den definierten Zeitintervallen zu dem Verhältnis der entsprechenden Keimzahlen zum Zeitpunkt der Beimpfung wird schließlich ein in log-Stufen ausgedrücktes Reduktionsverhältnis berechnet.

Zur Herstellung erfindungsgemäßer Feuchttücher wurde ein Tränkungsmittel auf einen Vliesstoff aufgebracht, der aus 30 Gew.-% Viskosefasern und 70 Gew.-% PET-Fasern besteht. Ein einzelnes Tuch hatte eine Masse von 46 g.

Bei dem Tränkungsmittel handelte es sich um eine Öl-in-Wasser-Emulsion, die gemäß der in Tabelle 1 angegebenen Rahmenrezeptur zusammengesetzt war:

**Tabelle 1: Rahmenrezeptur für O/W-Emulsion als Tränkungsmittel für erfindungsgemäße Feuchttücher**

| **Inhaltsstoff** | **Konzentration** / **[%]** |
|---|---|
| Wasser | ad 100 |
| Propylenglycol Dicaprylat/Dicaprat | 0,5 - 5,0 |
| Hydroxyacetophenon | 0,25 |
| Caprylyl Glycol | 0,10 - 1,00 |
| Propylene Glycol | 0,50 - 5,00 |
| Actives (z.B. Aloe Vera, Allantoin, Hyaluronsäure, Coenzym Q10, Tocopherol) | Jeweils 0,01 - 2,00 |
| Verdicker (z.B. Xanthan Gum, Acryl Polymer) | Jeweils 0,10 - 0,50 |
| pH-Regulierer (Citronensäure, Natriumhydroxid) | 0,01 - 1,00 |
| Öl (z.B. Mandelöl, Jojobaöl, Rapsöl etc.) | 0,10 - 3,00 |
| Natu rextrakte | 0,01 - 2,00 |
| Parfum | 0,1 -0,5 |

Das Tränkungsmittel wurde zu 4,04 g pro Gramm Vliesstoff auf letzteren aufgebracht.

Zur Durchführung des Konservierungsbelastungstests wurden Proben der auf diese Weise hergestellten erfindungsgemäßen Feuchttücher nun mit verschiedenen Mikroorganismen beimpft und anschließend bei 20-25 °C gelagert. Die Konzentration der jeweiligen Mikroorganismen in der Probe wurde jeweils zum Zeitpunkt der Beimpfung, sowie nach 2, 7, 14 und 28 Tagen bestimmt. Die Ergebnisse sind in der nachstehenden Tabelle 2 aufgeführt.

**Tabelle 2: Ergebnisse des Konservierungsbelastungstests**

| **Analyse** | **0 h** | **2 d** | **7 d** | **14 d** | **28 d** |
|---|---|---|---|---|---|
| Pseudomonas aeruginosa ATCC 9027 | 3,5 × 10⁵ | < 1,0 × 10¹ | < 1,0 × 10¹ | < 1,0 × 10¹ | < 1,0 × 10¹ |
| Log-Stufen | - | > 4,54 | > 4,54 | > 4,54 | > 4,54 |
| Staphylococcus aureus ATCC 6538 | 3,4 × 10⁵ | < 1,0 × 10¹ | < 1,0 × 10¹ | < 1,0 × 10¹ | < 1,0 × 10¹ |
| Log-Stufen | - | > 4,53 | > 4,53 | > 4,53 | > 4,53 |
| Escherichia coli ATCC 8739 | 9,0 × 10⁵ | < 1,0 × 10¹ | < 1,0 × 10¹ | < 1,0 × 10¹ | < 1,0 × 10¹ |
| Log-Stufen | - | > 4,95 | > 4,95 | > 4,95 | > 4,95 |
| Candida albicans ATCC 10231 | 3,7 × 10⁵ | < 1,0 × 10¹ | < 1,0 × 10¹ | < 1,0 × 10¹ | < 1,0 × 10¹ |
| Log-Stufen | - | > 4,57 | > 4,57 | > 4,57 | > 4,57 |
| Aspergillus brasiliensis ATCC 16404 | 4,2 × 10⁵ | < 7,2 × 10³ | < 1,0 × 10¹ | < 1,0 × 10¹ | < 1,0 × 10¹ |
| Log-Stufen | - | 1,77 | > 4,62 | > 4,62 | > 4,62 |

### Alle Angaben in koloniebildenden Einheiten / g

Wie den in Tab. 2 aufgeführten Ergebnissen zu entnehmen ist, ist die Keimbelastung nach der Beimpfung mit allen Mikroorganismen mit Ausnahme von *Aspergillus brasiliensis* bereits nach 2 Tagen auf den Endwert < 1,0 × 10¹ koloniebildende Einheiten pro Gramm gesunken. Im Falle der Beimpfung mit *Aspergillus brasiliensis* wird dieser Endwert nach 7 Tagen erreicht.

Für die Beurteilung der logarithmischen Reduktion werden grundsätzlich zwei verschiedene Akzeptanzkriterien, Kriterium A und Kriterium B, formuliert. Mit den o.g. Ergebnissen des Konservierungsbelastungstests erfüllt das erfindungsgemäße Feuchttuch beide dieser Kriterien.

### Beispiel 2: Konservierunasbelastunastest erfindunasaemäßer mit wässriaer Formulierung getränkter Feuchttücher

Zur Herstellung weiterer erfindungsgemäßer Feuchttücher wurde ein alternatives Tränkungsmittel auf einen aus Viskosefasern bestehenden Vliesstoff aufgebracht.

Bei dem Tränkungsmittel handelte es sich um eine wässrige Formulierung, die gemäß der in Tabelle 3 angegebenen Rahmenrezeptur zusammengesetzt war:

**Tabelle 3: Rahmenrezeptur für eine wässrige Lösung als Tränkungsmittel für erfindungsgemäße Feuchttücher**

| **Inhaltsstoff** | **Konzentration / [%]** | |
|---|---|---|
| Wasser | ad 100 | |
| Coco Glucoside | 0,10 - 1,50 | |
| Hydroxyacetophenon | 0,25 | |
| Caprylyl Glycol | 0,10 - 1,00 | |
| Propylene Glycol | 0,50 - 5,00 | |
| Actives (z.B. Aloe Vera, Allantoin, Hyaluronsäure, Coenzym Q10, Tocopherol) | 0,01 - 2,00 | |
| pH-Regulierer (Citronensäure, Natriumhydroxid) | 0,01 - 1,00 | |
| Naturextrakte | 0,01 - 2,00 | |
| Parfum | 0,1 -0,5 | |

Das Tränkungsmittel wurde zu 1,8 g pro Gramm Vliesstoff auf letzteren aufgebracht.

Zur Durchführung des Konservierungsbelastungstests wurden Proben der auf diese Weise hergestellten erfindungsgemäßen Feuchttücher nun mit verschiedenen Mikroorganismen beimpft und anschließend bei 20-25 °C gelagert. Die Konzentration der jeweiligen Mikroorganismen in der Probe wurde jeweils zum Zeitpunkt der Beimpfung, sowie nach 2, 7, 14 und 28 Tagen bestimmt. Die Ergebnisse sind in der nachstehenden Tabelle 4 aufgeführt.

**Tabelle 4: Ergebnisse des Konservierungsbelastungstests**

| **Analyse** | **0 h** | **2 d** | **7 d** | **14 d** | **28 d** |
|---|---|---|---|---|---|
| Pseudomonas aeruginosa ATCC 9027 | 2,1 × 10⁵ | < 1,0 × 10¹ | < 1,0 × 10¹ | < 1,0 × 10¹ | < 1,0 × 10¹ |
| Log-Stufen | - | > 4,32 | > 4,32 | > 4,32 | > 4,32 |
| Staphylococcus aureus ATCC 6538 | 3,3 × 10⁵ | < 1,0 × 10¹ | < 1,0 × 10¹ | < 1,0 × 10¹ | < 1,0 × 10¹ |
| Log-Stufen | - | > 4,52 | > 4,52 | > 4,52 | > 4,52 |
| Escherichia coli ATCC 8739 | 3,7 × 10⁵ | < 1,0 × 10¹ | < 1,0 × 10¹ | < 1,0 × 10¹ | < 1,0 × 10¹ |
| Log-Stufen | - | > 4,57 | > 4,57 | > 4,57 | > 4,57 |
| Candida albicans ATCC 10231 | 4,1 × 10⁵ | < 1,0 × 10¹ | < 1,0 × 10¹ | < 1,0 × 10¹ | < 1,0 × 10¹ |
| Log-Stufen | - | > 4,61 | > 4,61 | > 4,61 | > 4,61 |
| Aspergillus brasiliensis ATCC 16404 | 1,7 × 10⁵ | 1,3 × 10⁴ | 3,2 × 10⁴ | 6,0 × 10³ | 8,2 × 10¹ |
| Log-Stufen | - | 1,12 | 0,73 | 1,45 | 3,32 |

| | | | | | |
|---|---|---|---|---|---|
| Alle Angaben in koloniebildenden Einheiten / g | | | | | |

Wie den in Tab. 4 aufgeführten Ergebnissen zu entnehmen ist, ist die Keimbelastung nach der Beimpfung mit allen Mikroorganismen mit Ausnahme von *Aspergillus brasiliensis* bereits nach 2 Tagen auf den Endwert < 1,0 × 10¹ koloniebildende Einheiten pro Gramm gesunken.

Im Falle der Beimpfung mit *Aspergillus brasiliensis* sinkt die Keimbelastung erheblich langsamer und erreicht nach 28 Tagen einen Wert von 8,2 × 10¹ koloniebildende Einheiten pro Gramm.

Entsprechend den o.g. Ergebnissen des Konservierungsbelastungstests erfüllt das erfindungsgemäße Feuchttuch das Akzeptanzkriterium B.

### Beispiel 3: Prüfung der Hautverträglichkeit des Tränkunasmittels für erfindunasgemäße Feuchttücher

Zur Prüfung der Hautverträglichkeit des Tränkungsmittels, mit dem erfindungsgemäße Feuchttücher getränkt sind, wird ein Patch-Test an 50 Probanden durchgeführt. Mit einem Patch-Test können dermatologische und kosmetische Produkte auf ihr irritatives Potential untersucht werden. Bei den 50 ausgewählten Personen im Alter zwischen 18 und 69 Jahren handelte es sich um 28 Hautgesunde, 2 Atopiker, 2 Allergiker und 18 Personen mit empfindlicher Haut.

Für den Patch-Test wurde eine gemäß der in Tabelle 1 (Beispiel 1) angegebenen Rahmenrezeptur zusammengesetzte O/W-Emulsion für 48 Stunden unverdünnt auf den Rücken der Probanden mit Hilfe von quadratischen Kunststoffkammern (allergEAZE^{®} clear Patch Test Chambers; SmartPractice^{®}, Phoenix, AZ) unter Okklusion appliziert. Als Positivkontrolle diente der Modellschadstoff Natriumlaurylsulfat (SDS) in einer Konzentration von 1 % in Wasser. Als negative Kontrolle diente Wasser.

Die Bewertung der Testreaktionen erfolgte nach 48 Stunden (30 Minuten nach der Entfernung der Okklusion) sowie nach 72 Stunden, wobei die Haut der Probanden jeweils hinsichtlich Erythem, Schuppung und Fissuren untersucht wurde.

Die Testergebnisse zeigten, dass die 1%-ige SDS-Lösung bei 14 Testpersonen zu einer positiven Reaktion führte. Die negative Kontrolle zeigte erwartungsgemäß bei keiner Testperson eine Reaktion. Auch auf die erfindungsgemäße O/W-Emulsion zeigte keine Testperson eine Reaktion, weshalb dieses Tränkungsmittel hinsichtlich einer eventuell hautreizenden Wirkung als unbedenklich einzustufen ist.

### Beispiel 4: Prüfung der Eignung erfindungsgemäßer Feuchttücher als Gesichtsreinigungstücher

Erfindungsgemäße Feuchttücher, die mit einer gemäß der in Tabelle 1 (Beispiel 1) angegebenen Rahmenrezeptur zusammengesetzten O/W- Emulsion getränkt waren, wurden zur Prüfung ihrer Eignung als Gesichtsreinigungstücher von 20 augengesunden Probandinnen über einen Zeitraum von 14 Tagen getestet. Während des Testzeitraums wurden die Feuchttücher einmal täglich von den Probandinnen zur Augenmake-up Entfernung angewandt. Vor Beginn und nach Ende der 14-tägigen Anwendung wurde der vordere Augenabschnitt mit einem Spaltlampenmikroskop der Firma Rodenstock (Type RO5000, mit 16-facher Vergrößerung) inspiziert. Geprüft wurden folgende Parameter:
1) Beschaffenheit der Haut und Anhangsgebilde;
2) Beschaffenheit der palpebralen und bulbären Bindehaut;
3) Beschaffenheit der Hornhaut.

Als Ergebnis der Untersuchung konnte festgestellt werden, dass es in keinem Fall durch die Verwendung der erfindungsgemäßen Feuchttücher zu morphologisch sichtbaren krankhaften Veränderungen am vorderen Augenabschnitt kam, es erfolgte keine Veränderung der okulären und periokulären Befunde.

Die erfindungsgemäßen Feuchttücher eignen sich daher zur Gesichtsreinigung und insbesondere zur Entfernung von Augenmake-up. Sie sind im augenärztlichen Sinne als unbedenklich zu betrachten und können als augenverträglich bezeichnet werden.

### Beispiel 5: Weiterer Konservierungsbelastungstest erfindungsgemäßer mit wässriaer Formulierung getränkter Feuchttücher

Zur Herstellung weiterer erfindungsgemäßer Feuchttücher wurde ein wässriges Tränkungsmittel auf einen zu 30 % aus Viskosefasern und zu 70 % aus PET-Fasern bestehenden Vliesstoff aufgebracht.

Das Tränkungsmittel enthielt 0,5 Gew.-% Hydroxyacetophenon, wies einen pH-Wert von 6,0 auf, und enthielt keinen Puffer. Es wurde in einer Tränkungsmenge von 2,0 g Tränkungsmittel pro Gramm Vliesstoff auf diesen aufgebracht.

Zur Durchführung des Konservierungsbelastungstests wurden Proben der auf diese Weise hergestellten erfindungsgemäßen Feuchttücher nun mit verschiedenen Mikroorganismen beimpft und anschließend bei 20-25 °C gelagert. Die Konzentration der jeweiligen Mikroorganismen in der Probe wurde jeweils zum Zeitpunkt der Beimpfung, sowie nach 7, 14 und 28 Tagen bestimmt. Die Ergebnisse sind in der nachstehenden Tabelle 5 aufgeführt.

**Tabelle 5: Ergebnisse des Konservierungsbelastungstests**

| **Analyse** | **0 h** | **7 d** | **14 d** | **28 d** |
|---|---|---|---|---|
| Mischkeimsuspension Bakterien (P.aeruginosa ATCC 9027, Staph.aureus ATCC 6538, E.coli ATCC 8739) | 3,2 × 10⁵ | 2,8 × 10² | < 1,0 × 10¹ | < 1,0 × 10¹ |
| Log-Stufen | - | 3,06 | > 4,51 | > 4,51 |
| Mischkeimsuspension Hefen & Schimmelpilze (C.albicans ATCC 10231, A.brasiliensis ATCC 16404) | 2,0 × 10⁵ | 6,3 × 10³ | 3,8 × 10³ | < 1,0 × 10¹ |
| Log-Stufen | - | 1,50 | 1,72 | > 4,30 |

### Alle Angaben in koloniebildenden Einheiten / g

Entsprechend den in Tabelle 5 angegebenen Ergebnissen des Konservierungsbelastungstests erfüllt das erfindungsgemäße Feuchttuch das Akzeptanzkriterium B.

Der Konservierungsbelastungstest wurde nun erneut mit erfindungsgemäßen Feuchttüchern durchgeführt, deren Vliesstoff mit einem optimierten wässrigen Tränkungsmittel getränkt war. Das Tränkungsmittel enthielt ebenfalls 0,5 Gew.-% Hydroxyacetophenon, zusätzlich jedoch 0,25 Gew.-% Citratpuffer, der pH-Wert betrug 4,5. Es wurde mit einem Tränkungsgrad von 3,0 g pro Gramm Vliesstoff auf diesen aufgetragen. Anschließend wurde auf analoge Weise der Konservierungsbelastungstest durchgeführt, dessen Ergebnisse in der nachstehenden Tabelle 6 im Detail angegeben sind.

**Tabelle 6: Ergebnisse des Konservierungsbelastungstests**

| **Analyse** | **0 h** | **2 d** | **7 d** | **14 d** | **28 d** |
|---|---|---|---|---|---|
| Pseudomonas aeruginosa ATCC 9027 | 1,5 × 10⁵ | < 1,0 × 10¹ | < 1,0 × 10¹ | < 1,0 × 10¹ | < 1,0 × 10¹ |
| Log-Stufen | - | > 4,18 | > 4,18 | > 4,18 | > 4,18 |
| Staphylococcus aureus ATCC 6538 | 2,4 × 10⁵ | < 1,0 × 10¹ | < 1,0 × 10¹ | < 1,0 × 10¹ | < 1,0 × 10¹ |
| Log-Stufen | - | > 4,38 | > 4,38 | > 4,38 | > 4,38 |
| Escherichia coli ATCC 8739 | 7,6 × 10⁵ | < 1,0 × 10¹ | < 1,0 × 10¹ | < 1,0 × 10¹ | < 1,0 × 10¹ |
| Log-Stufen | - | > 4,88 | > 4,88 | > 4,88 | > 4,88 |
| Candida albicans ATCC 10231 | 2,5 × 10⁵ | 5,7 × 10⁴ | < 1,0 × 10¹ | < 1,0 × 10¹ | < 1,0 × 10¹ |
| Log-Stufen | - | 0,64 | > 4,40 | > 4,40 | > 4,40 |
| Aspergillus brasiliensis ATCC 16404 | 1,3 × 10⁵ | 2,1 × 10⁴ | < 1,0 × 10¹ | < 1,0 × 10¹ | < 1,0 × 10¹ |
| Log-Stufen | - | 0,79 | > 4,11 | > 4,11 | > 4,11 |

### Alle Angaben in koloniebildenden Einheiten / g

Durch den geringen Zusatz von Citratpuffer sowie durch die erhöhte Tränkmenge konnte der Grad der Keimzahlminderung noch erheblich erhöht werden, sodass die mit diesem Tränkungsmittel in dieser Tränkmenge getränkten Feuchttücher sogar das Akzeptanzkriterium A erfüllen.

Es konnte daher belegt werden, dass erfindungsgemäße Feuchttücher, die mit einem Tränkungsmittel versehen sind, das ausschließlich Hydroxyacetophenon als Inhaltsstoff mit konservierenden Eigenschaften enthält, hervorragende antimikrobielle Eigenschaften aufweisen und bei geeigneter Zusammensetzung und Konzentration des Tränkungsmittels sogar das den höchsten Anforderungen genügende Akzeptanzkriterium A im Konservierungsbelastungstest erreicht werden kann.

Die in der vorstehenden Beschreibung sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Feuchttuch zur Gesichts- und/oder Körperreinigung, als Anti-Aging-Mittel, als Anti-Pollution-Mittel, als Gesichtsmaske, zur Make-up-Entfernung, als Toilettenpapier, als Tuch zur Babyreinigung und/oder für die Kontaktierung und/oder Reinigung von Schleimhäuten und/oder Augenpartien, umfassend einen Vliesstoff und ein mehrere Inhaltsstoffe enthaltendes Tränkungsmittel, **dadurch gekennzeichnet, dass** das Tränkungsmittel ungepuffert ist und Hydroxyacetophenon als alleiniges Konservierungsmittel enthält.

2. Feuchttuch nach Anspruch 1, **dadurch gekennzeichnet, dass** das Tränkungsmittel ein Tensidsystem enthält, das Betaine und/oder Lauryl Glucosid umfasst oder daraus besteht.

3. Feuchttuch nach einem der voranstehenden Ansprüche, **dadurch gekennzeichnet, dass** der Vliesstoff aus Viskosefasern und/oder Lyocell-Fasern und/oder PET-Fasern besteht.

4. Feuchttuch nach einem der Ansprüche 1 bis 2, **dadurch gekennzeichnet, dass** der Vliesstoff aus Viskosefasern und/oder Lyocell-Fasern und/oder PET-Fasern und zusätzlich aus 0,1 - 20,0 Gew.-% Mikrofasern und/oder 0,1 - 30,0 Gew.-% Baumwoll-Fasern besteht.

5. Feuchttuch nach einem der voranstehenden Ansprüche, **dadurch gekennzeichnet, dass** der Vliesstoff 30 - 70 Gew.-% Viskosefasern und 30 - 70 Gew.-% PET-Fasern sowie optional 0,1 - 5,0 Gew.-% Mikrofasern und/oder 0,1 - 5,0 Gew.-% Baumwoll-Fasern umfasst oder daraus besteht.

6. Feuchttuch nach einem der voranstehenden Ansprüche, **gekennzeichnet durch** eine Tränkmenge von max. 4,0 g Tränkungsmittel pro Gramm Vliesstoff, wobei das Tränkungsmittel 0,125 Gew.-% bis 0,6 Gew.-% Hydroxyacetophenon sowie optional Betaine und/oder Lauryl Glucosid enthält.

7. Verfahren zur Herstellung eines Feuchttuches nach einem der Ansprüche 1 bis 6, umfassend das Aufbringen eines Tränkungsmittels auf einen Vliesstoff.

8. Verwendung eines Feuchttuches nach einem der Ansprüche 1 bis 6 zur Gesichts- und/oder Körperreinigung, als Anti-Aging-Mittel, als Anti-Pollution-Mittel, als Gesichtsmaske, zur Make-up-Entfernung, als Toilettenpapier und/oder als Tuch zur Babyreinigung oder für die Kontaktierung und/oder Reinigung von Schleimhäuten und/oder Augenpartien.

## Claims

1. Wet wipe for facial and/or body cleansing, as an anti-aging agent, as an anti-pollution agent, as a face mask, for make-up removal, as toilet paper, as a wipe for baby cleansing and/or for contacting and/or cleansing mucous membranes and/or eye areas, comprising a nonwoven fabric and an impregnating agent containing a plurality of ingredients, **characterized in that** the impregnating agent is unbuffered and contains hydroxyacetophenone as the sole preservative.

2. Wet wipe according to claim 1, **characterized in that** the impregnating agent contains a surfactant system comprising or consisting of betaine and/or lauryl glucoside.

3. Wet wipe according to one of the preceding claims, **characterized in that** the nonwoven fabric consists of viscose fibers and/or lyocell fibers and/or PET fibers.

4. Wet wipe according to one of the claims 1 to 2, **characterized in that** the nonwoven fabric consists of viscose fibers and/or lyocell fibers and/or PET fibers and additionally of 0.1-20.0% by weight of microfibers and/or 0.1 - 30.0% by weight of cotton fibers.

5. Wet wipe according to one of the preceding claims, **characterized in that** the nonwoven fabric comprises 30 - 70% by weight viscose fibers and 30 - 70% by weight PET fibers and optionally 0.1 - 5.0% by weight microfibers and/or 0,1-5.0% by weight of cotton fibers or consists thereof.

6. Wet wipe according to one of the preceding claims, **characterized by** a maximum impregnation quantity of 4.0 g of impregnating agent per gram of nonwoven fabric, the impregnating agent containing 0.125% by weight to 0.6% by weight of hydroxyacetophenone and optionally betaines and/or lauryl glucoside.

7. Process for production of a wet wipe according to one of the claims 1 to 6, comprising applying an impregnating agent onto a nonwoven fabric.

8. Use of a wet wipe according to one of the claims 1 to 6 for facial and/or body cleansing, as an anti-aging agent, as an anti-pollution agent, as a face mask, for make-up removal, as toilet paper and/or as a tissue for baby cleansing or for contacting and/or cleansing mucous membranes and/or eye areas.

## Revendications

1. Lingette humide destinée au nettoyage du corps et/ou du visage, en tant que produit anti-âge, en tant que produit antipollution, en tant que masque visage, destinée au démaquillage, en tant que papier toilette, en tant que lingette destinée au nettoyage de bébés et/ou pour la mise en contact avec les et/ou le nettoyage des muqueuses et/ou parties oculaires, comprenant un non-tissé et un agent d'imprégnation contenant plusieurs composants, **caractérisée en ce que** l'agent d'imprégnation est non tamponné et contient de l'hydroxyacétophénone en tant que seul conservateur.

2. Lingette humide selon la revendication 1, **caractérisée en ce que** l'agent d'imprégnation contient un système tensioactif qui comprend ou consiste en des bétaïnes et/ou du lauryl glucoside.

3. Lingette humide selon l'une quelconque des revendications précédentes, **caractérisée en ce que** le non-tissé est constitué de fibres de viscose et/ou fibres de Lyocell et/ou fibres de PET.

4. Lingette humide selon l'une quelconque des revendications 1 et 2, **caractérisée en ce que** le non-tissé est constitué de fibres de viscose et/ou fibres de Lyocell et/ou fibres de PET et en outre de 0,1 - 20,0 % en poids de microfibres et/ou 0,1 - 30,0 % en poids de fibres de coton.

5. Lingette humide selon l'une quelconque des revendications précédentes, **caractérisée en ce que** le non-tissé comprend ou consiste en 30 - 70 % en poids de fibres de viscose et 30 - 70 % en poids de fibres de PET ainsi qu'en option 0,1 - 5,0 % en poids de microfibres et/ou 0,1 - 5,0 % en poids de fibres de coton.

6. Lingette humide selon l'une quelconque des revendications précédentes, **caractérisée par** une quantité d'imprégnation d'au maximum 4,0 g d'agent d'imprégnation par gramme de non-tissé, l'agent d'imprégnation contenant 0,125 % en poids à 0,6 % en poids d'hydroxyacétophénone ainsi qu'en option des bétaïnes et/ou du lauryl glucoside.

7. Procédé pour la fabrication d'une lingette humide selon l'une quelconque des revendications 1 à 6, comprenant l'application d'un agent d'imprégnation sur un non-tissé.

8. Utilisation d'une lingette humide selon l'une quelconque des revendications 1 à 6 pour le nettoyage du corps et/ou du visage, en tant que produit anti-âge, en tant que produit antipollution, en tant que masque visage, pour le démaquillage, en tant que papier toilette et/ou en tant que lingette destinée au nettoyage de bébés et/ou pour la mise en contact avec les et/ou le nettoyage des muqueuses et/ou parties oculaires.
